# EUROPEAN PATENT SPECIFICATION

(11) **EP 2 051 694 B1**
(45) Date of publication and mention of the grant of the patent: **18.10.2017**
(21) Application number: 07835588.0
(22) Date of filing: 15.08.2007
(51) Int. Cl.: A61K 9/00, A61K 9/20, A61K 31/4704, A61K 31/496, A61K 47/00

(54) **PHARMACEUTICAL COMPOSITIONS COMPRISING ARIPIPRAZOLE**
PHARMAZEUTISCHE ZUSAMMENSETZUNGEN MIT ARIPIPRAZOL
COMPOSITIONS PHARMACEUTIQUES COMPRENANT DE L'ARIPIPRAZOLE

(30) Priority: 15.08.2006 TR 200604349
(43) Date of publication of application: 29.04.2009
(73) Proprietor: Nobel Ilaç Sanayii Ve Ticaret A.S., 34768 Umraniye/Istanbul (TR)
(72) Inventor: USLU, Abdullah, 34353 Istanbul (TR)
(86) International application number: PCT/TR2007/000076
(87) International publication number: WO 2008/020820

(56) References cited:
- EP-A- 1 808 164
- EP-A- 1 808 165
- WO-A-03/030868
- WO-A-2005/041937
- WO-A-2006/097344
- US-A1- 2005 019 398

## Description

### Technical Field

This invention relates to pharmaceutical compositions comprising aripiprazole and/or its pharmaceutically acceptable salts and preparation method of these pharmaceutical compositions.

### Background Art

Aripiprazole, 7-{4-[4-(2,3-Dichlorophenyl)-1-piperazinyl]butyloxy}-3,4-dihydrocarbostyrl and its use for the treatment of schizophrenia as an atypical antipsychotic agent was disclosed in U.S. patent No 5,006,528. The drugs that comprise aripiprazole are approved in many countries with trendmark ABILIFY^{®} and bring in usage of the patients. ABILIFY^{®} tablet formulation includes starch, hydroxypropyl cellulose, lactose monohydrate, magnesium stearate and microcrystalline cellulose as supportive agent.

According to Japanese Unexamined Patent Publication No.191256/1990, anhydrous aripiprazole crystals are manufactured and obtained by the recrystallization step with ethanol.

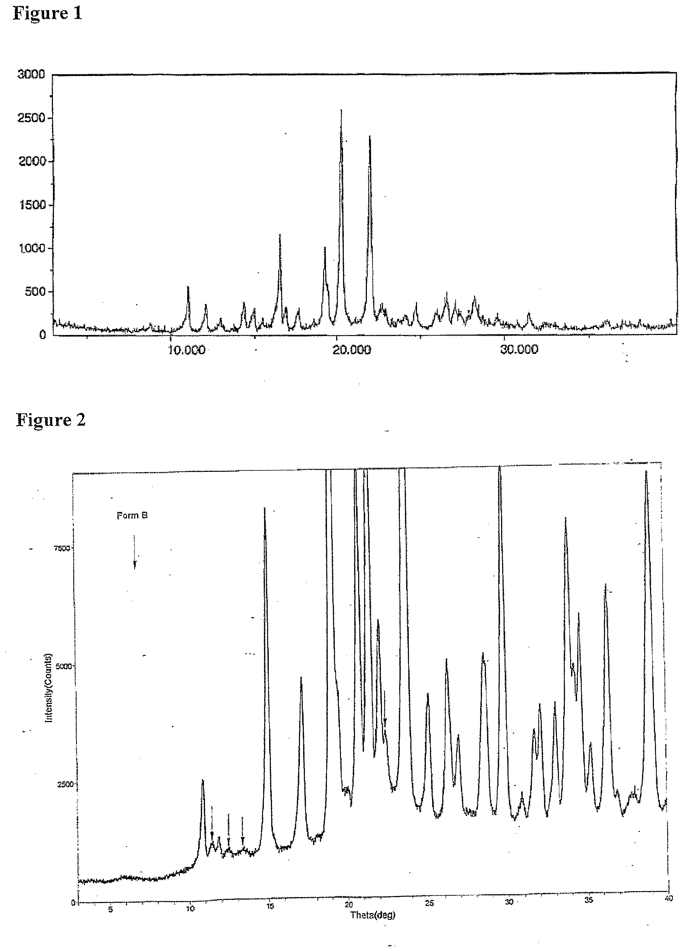

Also, according to the Proceedings of the "4th Japanese-Korean Symposium on Separation Technology" symposium (October 6-8, 1996), anhydrous aripiprazole crystals are manufactured by heating aripiprazole hydrate at 80°C. The product obtained by this method is named as aripiprazole Type 1 crystal:
The XRD graphs of aripiprazole crystals obtained by this method are shown in Figure 1.

Anhydrous aripiprazole crystals obtained by both methods have high hygroscopic property and when exposed to moisture, the anhydrous form can take on water and convert to hydrous form. The hydrous forms of aripiprazole are less bioavailable and less dissoluble than the anhydrous forms of aripiprazole. In addition, usage of the hydrous aripiprazole crystals in the production of medicine manufacture causes breakdown in production processes and increases the procurement costs. Additionally, the shelf-life of the high hydroscopic aripiprazole crystals could be significantly decreased.

The negations explained above can cause a decrease in quality of the end product, consequently problems in the approvement procedures. Pharmaceutical products which have improved shelf-life, suitable bioavailability and dissolution levels are needed to solve these problems.

To compensate these needs, a new formulation comprising known aripiprazole crystals that stave off the problems above should be improved or the new form of aripiprazole crystal should be applied to the known formulation.

The PCT application No.WO 2003/026659 explains the new aripiprazole crystals named aripiprazole Form B. This patent application aims the manufacture of high hygroscopic aripiprazole forms that have improved shelf-life, suitable bioavailability and dissolution levels and defines the production of high hygroscopic aripiprazole crystals. Anhydrous aripiprazole crystals of the present invention is characterized by maintaining water content of 0.4% or less after 24 hours inside a dessicator set at a temperature of 60°C and a humidity of 100%. The aripiprazole crystals characterized by patent number WO 2003/026659 have peaks at 2θ = 11.0°, 16.6°, 19.3°, 20.3°, 22.1°. These values are in accordance with the 2θ values of aripiprazole Type 1 crystals.

The PCT applications WO 2005016262, WO 2004064752 and WO 2004017897 state the aripiprazole enjectable formulations, WO 03030868 numbered PCT application defines flash-melt formulation and WO 02085366 explains oral solution formulation.

The patents of the known technique do not explain new high hygroscopic, anhydrous aripiprazole crystal tablet formulations higher then 0.4%.

### Objective of the Invention:

The objective of this invention is to provide a new tablet formulation that comprises anhydrous aripiprazole crystals having high hygroscopicity and has suitable stability value.

### Description of the Drawings :

Figure 1, shows the X-Ray peaks of aripiprazole Type 1 crystals.
Figure 2, shows the X-Ray peaks of the protected Form B tablet formulation produced by present invention after 3-month stability studies.

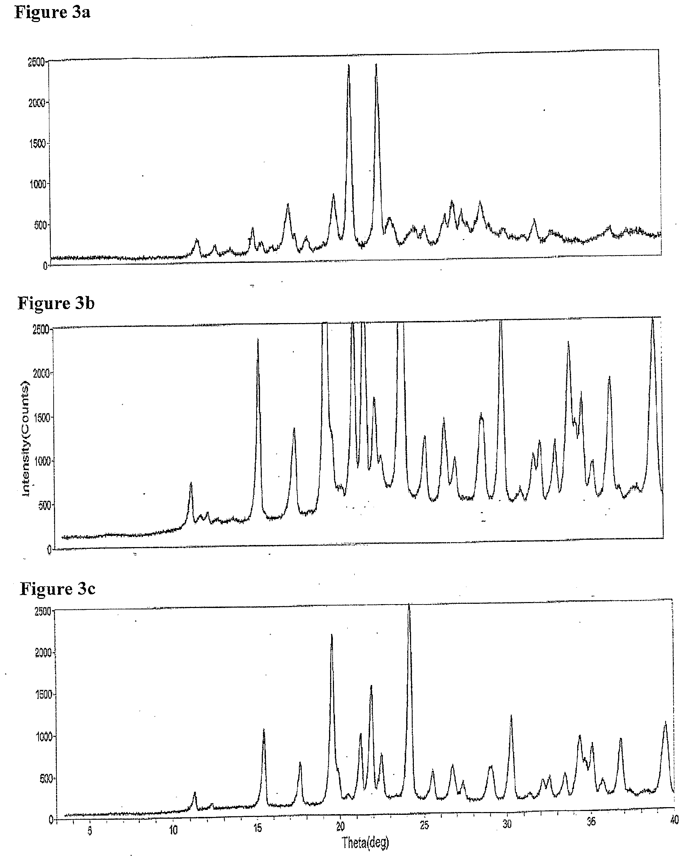

Figure 3a, shows the X-Ray peaks of the hygroscopic dry aripiprazole crystals used in present invention formulation.
Figure 3b, shows the X-Ray peaks of the tablets produced by present invention formulation after 3-month stability studies.
Figure 3c, shows the X-Ray peaks of the placebo tablets produced by present invention formulation after 3-month stability studies.
Figure 4, shows the dissolution profiles relative to ABILIFY^{®} produced by the formulation explained in Example 1 and Example 2.

### Detailed Description of the Invention:

The present invention which have suitable dissolution and stability values comprises aripiprazole or pharmaceutical acceptable salt, at least one filling material, at least one disintegrant, at least one binder, at least one lubricant and if desired optionally one coloring agent.

Present invention comprises mannitol, isomalt, sorbitol or mixture of these as filling material, crospovidone preferably thin, super thin or micronized crospovidone (Kollidon CL^{®}, Kollidon CL-F^{®}, Kollidon CL-SF^{®}, Kollidon CL-M^{®}), croscarmellose Sodium, carmellose Sodium, Sodium Starch Glycolate or mixture of these as disintegrant, Polyvinyl pyrolidone (PVP) as binder and magnesium stearate as lubricant.

In the preferred embodiment of the invention, filling material forms 50-80 %, disintegrant 1-20 %, binder 1-5 % and lubricant 0.3-2 % of the total tablet weight.

In the tablet formulation of the present invention, Type 1 crystals anhydrous aripiprazole crystals with 2θ values of 11.0°, 16.6°, 19.3°, 20.3°, 22.1° are used.

In the preferred embodiment of the invention, anhydrous Aripiprazole Crystals B having high hygroscopicity wherein said high hygroscopicity is a moisture content higher than 0,40 % after placing said drug substance for 24 hours in desiccator maintained at a temperature of 60°C and a humidity level of 100 % are used as an active ingredient.

The tablet formulation of the present invention which comprises aripiprazole and has suitable dissolution and stability values is explained in Example 1.

### Example 1

Aripiprazole 30 mg tablet formulation

| **Unit Formula** | **Amount** |
|---|---|
| Aripiprazole | 30 mg |
| Mannitol | 221,85 mg |
| Kollidon CL**-**M^{®} | 40 mg |
| PVPK 30 | 5 mg |
| FD&C Blue 2 (Lake Indigo Carmine) | 0,15 mg |
| Magnesium Stearate | 3 mg |
| Deionized water* | 0,05 ml |
| Total | 300 mg |

The tablet formulation explained above is produced as below.
- Half of the aripiprazole, mannitol and kollidon CL-M and half of the FD & C Blue are sifted from sifter, mix and pour to cauldron.
- PVPK 30 solution in deionized water is prepared as granulation solution.
- The granulation solution is sprayed on to the powder mixture above.
- The obtained dough is sifted.
- Dry granules are put through the dry stirring cauldron and the rest of CL-M and FD & C Blue 2 is added and mixed 10 minutes.
- Sifted magnesium stearate is added to the powder mixture prepared above, mixed 5 more minutes and tablets are pressed.

The tablets produced among the formulation explained in Example 1 comprise anhydrous aripiprazole crystals having high hygroscopicity.

### Example 2

In order to show the unexpected dissolution values of the present invention, tablets comprising anhydrous aripiprazole crystals having high hygroscopicity and excipients explained by ABILIFY^{®} was prepared. This production is achieved through the method explained in Example 18 of WO 2003/026659 numbered patent.

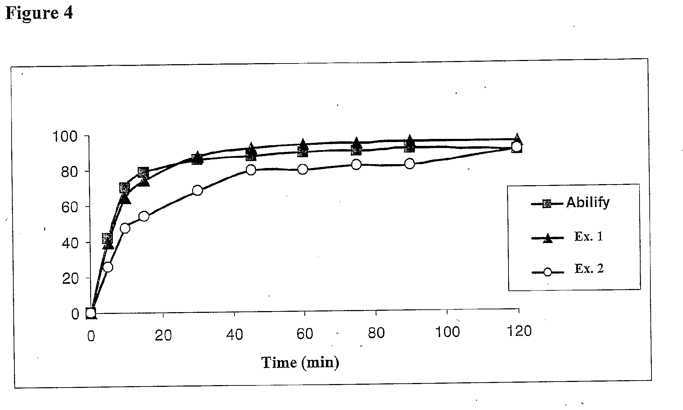

The comparison of the dissolution values of the tablets prepared according to Example 1 and Example 2 with the ABILIFY^{®} are listed in Table 1 and its profiles are shown in Figure 4.

When the hygroscopic aripiprazole crystals are produced by the present invention formulation applied from orally, it is seen from Table 1 that it solves the problems of the known technique and shows very similar diffusion profile with ABILIFY^{®}. The values on Table 1 are provided at pH=4,5 (USP Acetate Buffer), 75 RPM; Pedal; 900 ml environmental conditions.

**Table 1**

| **X Axis** | **Y1 Axis** | **Y2 Axis** | **Y3 Axis** |
|---|---|---|---|
| **Time (min.)** | **Abilify^{®} 30 mg Tablet** | **Aripiprazole 30 mg Tablet Example 1** | **Aripiprazole 30 mg Tablet Example 2** |
| 0 | 0 | 0 | 0 |
| 5 | 41,37 | 39,41 | 25,44 |
| 10 | 70,33 | 64,67 | 47,32 |
| 15 | 78,73 | 74,26 | 54,18 |
| 30 | 86,02 | 87,29 | 68,42 |
| 45 | 87,81 | 91,64 | 79,77 |
| 60 | 89,41 | 94,08 | 79,50 |
| 75 | 90,34 | 94,55 | 82,06 |
| 90 | 91,60 | 94,85 | 81,96 |
| 120 | 89,78 | 94,83 | 90,69 |

After 3-month stability studies, X-Ray peaks of the protected Type 1 anhydrous aripiprazole crystal tablets, produced by the present invention are shown in Figure 2. In other words, the stability problems coming up from the tablets comprising anhydrous aripiprazole crystals having high hygroscopicity are solved by the present invention formulation.

The XRD measurements shown in Figure 2 are achieved by Rigaku MiniFlex type copper tube X-Ray diffractometer. The diffractometer has solid state detector and glass plate with cavity. Diffractograms obtained are measured between 2-40 degrees and 0.05 degree interval. Mentioned diffractometer has continuous scanning property with scan speed 2 degrees / minute.

## Claims

1. A pharmaceutical tablet composition comprising;
a) high-hygroscopic anhydrous aripiprazole wherein said high hygroscopicity is a moisture content higher than 0,40% after placing said drug substance for 24 hours in desiccator maintained at a temperature of 60°C and a humidity level of 100% or a pharmaceutical acceptable salt thereof,
b) at least a filler selected from the group consisting of mannitol, isomalt and sorbitol,
c) at least a disintegrant selected from the group consisting of crospovidone croscarmellose sodium and sodium starch glycolate,
d) a pharmaceutical acceptable binder and
e) a pharmaceutical acceptable lubricant.

2. A pharmaceutical tablet composition according to claim 1, wherein the filler is mannitol.

3. A pharmaceutical tablet composition according to any one of claims 1 and 2, wherein the disintegrant is crospovidone.

4. A pharmaceutical tablet composition according to claim 3, wherein the crosspovidone is selected from group consisting of thin crosspovidone, super thin crospovidone and micronized crospovidone.

5. A pharmaceutical tablet composition according to claim 4, wherein the crospovidone is micronized crospovidone preferably Kollidon CL-M^{®}.

6. A pharmaceutical tablet composition according to any one of the claims 1 to 5, wherein the binder is PVP.

7. A pharmaceutical tablet composition according to claim 6, wherein the PVP is PVP K30.

8. A pharmaceutical tablet composition according to any one of the claims 1 to 7, wherein the lubricant is magnesium stearate.

## Patentansprüche

1. Eine pharmazeutische Tablettenzusammensetzung, umfassend;
a) hochhygroskopisches wasserfreies Aripiprazol, wobei die hohe Hygroskopizität ein Feuchtigkeitsgehalt von mehr als 0,40% ist, nachdem die Arzneistoffsubstanz für 24 Stunden bei einer Temperatur von 60 °C und einem Feuchtigkeitsgehalt von 100% in einem Exsikkator gehalten wird oder ein pharmazeutisch verträgliches Salz davon,
b) mindestens einen Füllstoff, ausgewählt aus der Gruppe bestehend aus Mannit, Isomalt und Sorbit,
c) mindestens ein Sprengmittel, ausgewählt aus der Gruppe bestehend aus Crospovidon, Croscarmellose-Natrium und Natriumstärke-Glykolat
d) ein pharmazeutisch verträgliches Bindemittel und
e) ein pharmazeutisch verträgliches Schmiermittel.

2. Eine pharmazeutische Tablettenzusammensetzung nach Anspruch 1, wobei der Füllstoff Mannit ist.

3. Eine pharmazeutische Tablettenzusammensetzung nach einem der Ansprüche 1 und 2, wobei das Sprengmittel Crosspovidon ist.

4. Eine pharmazeutische Tablettenzusammensetzung nach Anspruch 3, wobei das Crosspovidon ausgewählt ist aus der Gruppe bestehend aus dünnem Crosspovidon, superdünnem Crosspovidon und mikronisiertem Crosspovidon

5. Ein pharmazeutische Tablettenzusammensetzung nach Anspruch 4, wobei das Crosspovidon mikronisiertes Crosspovidon, vorzugsweise Kollidon CL-M^{®} ist.

6. Eine pharmazeutische Tablettenzusammensetzung nach einem der Ansprüche 1 und 5, wobei das Bindemittel PVP ist.

7. Eine pharmazeutische Tablettenzusammensetzung nach Anspruch 6, wobei das PVP, PVP K30 ist.

8. Eine pharmazeutische Tablettenzusammensetzung nach einem der Ansprüche 1 und 7, wobei das Schmiermittel Magnesiumstearat ist.

## Revendications

1. Composition de comprimé pharmaceutique comprenant :
a) L'aripiprazole anhydre hautement hygroscopique dans lequel ladite hygroscopicité élevée est une teneur en humidité supérieure à 0,40% après avoir placé ladite substance médicamenteuse pendant 24 heures dans un dessiccateur maintenu à une température de 60°C et un taux d'humidité de 100% ou un produit pharmaceutique acceptable sel de celui-ci,
b) Au moins une charge choisie dans le groupe constitué par le mannitol, l'isomalt et le sorbitol,
c) Au moins un désintégrant choisi dans le groupe consistant en crospovidone, croscarmellose sodium et amidon de sodium glycolate,
d) Un liant pharmaceutiquement acceptable et
e) Un lubrifiant pharmaceutiquement acceptable.

2. Composition de comprimé pharmaceutique selon la revendication 1, dans laquelle la charge est du mannitol.

3. Composition de comprimé pharmaceutique selon l'une quelconque des revendications 1 et 2, dans laquelle le désintégrant est la crosspovidone.

4. Composition de comprimé pharmaceutique selon la revendication 3, dans laquelle la crosspovidone est choisie dans le groupe constitué par la crosspovidone fine, la crosspovidone super fine et la crosspovidone micronisée.

5. Composition de comprimé pharmaceutique selon la revendication 4, dans laquelle la crospovidone est une crospovidone micronisée, de préférence Kollidon CL-M^{®}.

6. Composition de comprimé pharmaceutique selon l'une quelconque des revendications 1 et 5, dans laquelle le liant est un PVP.

7. Composition de comprimé pharmaceutique selon la revendication 6, dans laquelle la PVP est PVP K30.

8. Composition de comprimé pharmaceutique selon l'une quelconque des revendications 1 et 7, dans laquelle le lubrifiant est le stéarate de magnésium.
